# EUROPEAN PATENT SPECIFICATION

(11) **EP 4 419 510 B1**
(45) Date of publication and mention of the grant of the patent: **08.10.2025**
(21) Application number: 22808665.8
(22) Date of filing: 19.10.2022
(51) Int. Cl.: C07D 295/096, A61P 9/00, A61P 9/10, A61K 31/495

(54) **NOVEL TRIMETAZIDINE SALTS**
NEUE TRIMETAZIDINSALZE
NOUVEAUX SELS DE TRIMETAZIDINE

(30) Priority: 20.10.2021 EP 21306465
(43) Date of publication of application: 28.08.2024
(73) Proprietor: Les Laboratoires Servier, 92284 Suresnes Cedex (FR)
(72) Inventor: MAHIEUX, Julien, 45140 Ormes (FR); VILLARD, Frédéric, 45000 Orléans (FR)
(74) Representative: Giudicelli, Cathy
(86) International application number: PCT/EP2022/079054
(87) International publication number: WO 2023/066971

(56) References cited:
- WO-A1-2014/180240
- CN-A- 110 054 599
- CN-A- 110 105 307
- CN-A- 110 183 398

## Description

The present invention relates to novel trimetazidine salts, their hydrates, and crystalline forms thereof.

Trimetazidine, or 1-(2,3,4-trimethoxybenzyl)piperazine (formula (I)), is a compound which, by maintaining the energy metabolism of a cell exposed to hypoxia or ischaemia, avoids the collapse of the intracellular level of adenosine triphosphate (ATP). It thus ensures functioning of the ion pumps and sodium-potassium transmembrane flows and maintains cellular homeostasis.

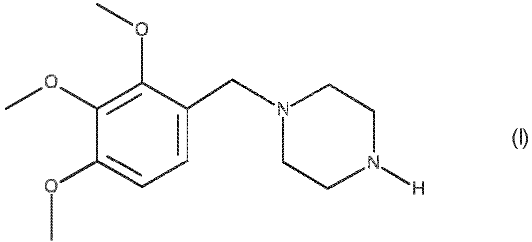

Trimetazidine dihydrochloride is currently used therapeutically for the prophylactic treatment of angina pectoris crisis, in chorioretinal attacks and for the treatment of vertigo of vascular origin (Ménière's vertigo, tinnitus).

Trimetazidine nitrosamine of formula (II) is an impurity which may be formed during the process of synthesis of trimetazidine salts or during the formulation process of such salts, in presence of nitrites coming from water and/or excipients.

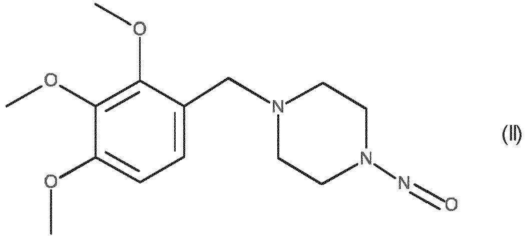

The Applicant has now found that the trimetazidine salts of the present invention allow to reduce the formation of trimetazidine nitrosamine in presence of nitrites.

The patent applications CN110105307, CN110183398 and CN110054599 respectively disclose the synthesis of the trimetazidine hemioxalate, oxalate and dioxalate salts, in order to overcome flowability, poor tableting property and hygroscopicity issues of the trimetazidine dihydrochloride salt.

The present invention relates especially to trimetazidine hemimalate, hemiadipate, hemitartrate, hemiphosphate, hemisulfate, hemisuccinate and hemifumarate salts.

The present invention also relates to a process of synthesis of trimetazidine salts, wherein trimetazidine is reacted with an organic acid in a solvent, leading to the corresponding salt having a trimetazidine/organic acid ratio of 2/1,
wherein the organic acid is selected from malic acid, adipic acid, tartaric acid, phosphoric acid, sulfuric acid, succinic acid, and fumaric acid.

In an embodiment of the present invention, trimetazidine is reacted with 0.5 eq. malic acid, adipic acid, tartaric acid, phosphoric acid, sulfuric acid, succinic acid or fumaric acid, in a solvent, leading to the corresponding trimetazidine hemimalate, hemiadipate, hemitartrate, hemiphosphate, hemisulfate, hemisuccinate or hemifumarate having a trimetazidine/ malic acid, trimetazidine/ adipic acid, trimetazidine/ tartaric acid, trimetazidine/ phosphoric acid, trimetazidine/ sulfuric acid, trimetazidine/ succinic acid or trimetazidine/ fumaric acid molar ratio of 2/1.

Among the solvents there may be mentioned more especially anisole, methyl isobutyl ketone, toluene, n-heptane, acetonitrile, methyl ethyl ketone, ethyl acetate, 1,3-dioxalane, tetrahydrofuran, acetone, methyl tert-butyl ether, water, alcohols such as methanol, ethanol, isopropanol, isobutanol or n-butanol, and mixtures thereof.

The salification reaction is preferably performed at a temperature of 20°C to 70°C.

The trimetazidine salts of the present invention allow to reduce the formation of trimetazidine nitrosamine in presence of nitrites.

According to an embodiment of the present invention, the trimetazidine salts of the present invention form less than 1 ppm of trimetazidine nitrosamine in a solution containing nitrites, after 22h at 40°C.

The present invention also relates to pharmaceutical compositions comprising a trimetazidine hemimalate, hemiadipate, hemitartrate, hemiphosphate, hemisulfate, hemisuccinate or hemifumarate salt in combination with one or more inert, non-toxic, pharmaceutically acceptable excipients or carriers.

The useful dosage varies according to the age and weight of the patient, the administration route, the nature and severity of the disorder and any associated treatments, and ranges from 15 mg to 200 mg per day in one or more administrations.

Among the pharmaceutical compositions according to the invention there may be mentioned more especially those that are suitable for oral, parenteral (intravenous, intramuscular or subcutaneous), per- or trans-cutaneous, nasal, rectal, perlingual, ocular or respiratory administration, and especially tablets or dragées, sublingual tablets, gelatin capsules, capsules, minigranules, suppositories, creams, ointments, dermal gels, injectable or drinkable preparations, aerosols, and eye or nasal drops.

The pharmaceutical compositions may be in the form of immediate release or prolonged release compositions.

According to one aspect of the present invention, the pharmaceutical composition is a tablet for immediate release oral administration.

According to another aspect of the present invention, the pharmaceutical composition is a matrix tablet for prolonged release oral administration.

According to another aspect of the present invention, the pharmaceutical composition is in the form of coated minigranules in capsules, for oral, once-a-day administration.

In addition to the trimetazidine salt, the tablets according to the invention comprise one or more excipients or carriers, such as diluents, retardants, lubricants, binders, disintegrators, absorbents, plasticizers, colourants and sweeteners.

There may be mentioned as examples of excipients or carriers:
◆ *for the diluents:* lactose, dextrose, sucrose, mannitol, sorbitol, glycerol, calcium hydrogen phosphate dihydrate, calcium carbonate, cellulose, cellulose ethers such as hydroxypropylcellulose, hydroxyethylcellulose, hydroxymethylcellulose, methylcellulose or hydroxypropyl methylcellulose,
◆ *for the controlled-release agent:* ethylcellulose, ethylcellulose derivatives such as cellulose acetate, cellulose acetate butyrate, cellulose acetate propionate, cellulose acetate phthalate, hydroxypropyl methylcellulose acetate succinate and/or polymethacrylates,
◆ *for the lubricants:* silica, talc, stearic acid and its magnesium and calcium salts, polyethylene glycol, glycerol behenate or sodium benzoate,
◆ *for the binders:* aluminium and magnesium silicate, starch, gelatin, tragacanth, methylcellulose, sodium carboxymethylcellulose and polyvinylpyrrolidone,
◆ *for the plasticizers:* acetyl tributyl citrate, glycerol triacetate, acetyl triethyl citrate, acetyl ethyl citrate, diethyl sebacate, dibutyl sebacate, ethyl phthalate, dibutyl phthalate, polyethylene glycol, glycerol and/or propylene glycol,
◆ *for the disintegrants:* agar, alginic acid and its sodium salt, effervescent mixtures.

The percentage of active ingredient of formula (I) in the tablet is preferably between 5% and 50% by weight.

According to one aspect of the present invention, the trimetazidine salt according to the present invention is administered in association with one or more additional active ingredients.

The administration in association may be in the form of a simultaneous or successive coadministration of two or more separate pharmaceutical compositions each containing one of the active ingredients (free association), or in the form of the administration of a fixed-dose combination including two or more active ingredients combined in a single dosage form.

Among the additional active ingredients, there may be mentioned more especially betablockers.

According to one aspect of the present invention, the trimetazidine salt of the present invention is administered in association with a betablocker in the form of a fixed-dose combination. Among the betablockers, there may be mentioned more especially metoprolol and bisoprolol. According to a preferred aspect of the present invention, the fixed-dose combination of the trimetazidine salt and bisoprolol is a capsule containing 80 mg trimetazidine salt minigranules and 5 or 10 mg bisoprolol hemifumarate pellets.

According to another preferred aspect of the present invention, the fixed-dose combination of the trimetazidine salt and metoprolol is a capsule containing 80 mg trimetazidine salt minigranules, and 47.5 or 95 mg metoprolol succinate pellets.

A formulation of bisoprolol hemifumarate pellets is already disclosed in Example 4a of WO2016/071631.

### ABBREVIATIONS

- eq.: molar equivalent
- NMR: Nuclear Magnetic Resonance
- XRPD: X-Ray Powder Diffraction

### EXPERIMENTAL PART

For the compounds of Examples 1, 3, 4, 5 and 7, the XRPD patterns were recorded from 3.5 °2θ to 35°2θ using an X-ray diffractometer operating in the transmission mode with CuKα radiation (λ = 1.5418Å) at 45kV and 40 mA and with a 0.013 °2θ step size for 10 minutes.

For the compounds of Examples 2 and 6, the XRPD patterns were recorded from 3.5 °2θ to 55°2θ using an X-ray diffractometer operating in the transmission mode with CuKα radiation (λ = 1.5418Å) at 45kV and 40 mA and with a 0.013 °2θ step size for 30 minutes.

Only the peaks with a relative intensity higher than 10% have been mentioned in the XRPD tables.

The chemical shifts, in ppm, are given with respect to tetramethylsilane (TMS), using partially deuterated dimethylsulfoxyde or partially deuterated methanol as internal standard.

In solution in partially deuterated dimethylsulfoxyde, the resonance at 2.5 ppm on the 1D 1H NMR spectrum is due to partially deuterated dimethylsulfoxyde and the resonance at 3.30 ppm is due to the presence of water.

In solution in partially deuterated methanol, the resonance at 3.3 ppm on the 1D 1H NMR spectrum is due to partially deuterated methanol and the resonance at 4.83 ppm is due to the presence of water.

### EXAMPLE 1 : Trimetazidine hemiadipate

1.0 g of trimetazidine and 0.28g (0.5 eq) of adipic acid were placed in 4mL of isopropanol. The mixture was placed under stirring for 22h at ambient temperature. The suspension was filtered, and the powder was dried at 40°C under vacuum overnight. The obtained powder corresponds to a crystalline powder.

¹H NMR: 6,95 ppm, 6.75 ppm, 3.77 ppm, 3.76 ppm, 3.73 ppm, 3.33 ppm, 2.67 ppm, 2.29 ppm, 2.18 ppm, 1.49 ppm.

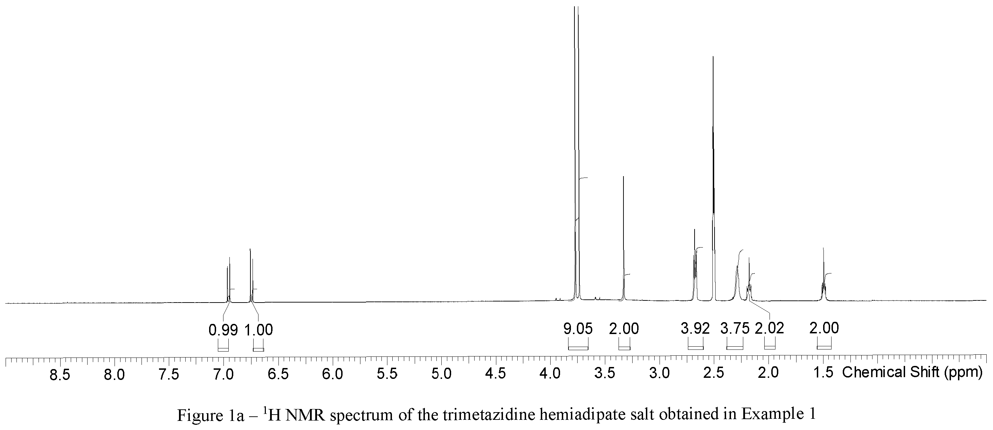

The corresponding ¹H NMR spectrum is in Figure 1a.

**XRPD table:**

| | Position [°2θ] | Relative Intensity [%] |
|---|---|---|
| 1 | 8.64 | 31 |
| 2 | 12.17 | 12 |
| 3 | 12.32 | 20 |
| 4 | 13.74 | 15 |
| 5 | 14.64 | 12 |
| 6 | 15.65 | 11 |
| 7 | 15.95 | 27 |
| 8 | 16.81 | 20 |
| 9 | 17.35 | 12 |
| 10 | 18.15 | 100 |
| 11 | 19.33 | 33 |
| 12 | 19.57 | 28 |
| 13 | 20.54 | 11 |
| 14 | 21.91 | 11 |
| 15 | 22.29 | 12 |
| 16 | 22.99 | 56 |
| 17 | 23.77 | 21 |
| 18 | 24.48 | 19 |
| 19 | 26.15 | 11 |
| 20 | 27.49 | 15 |

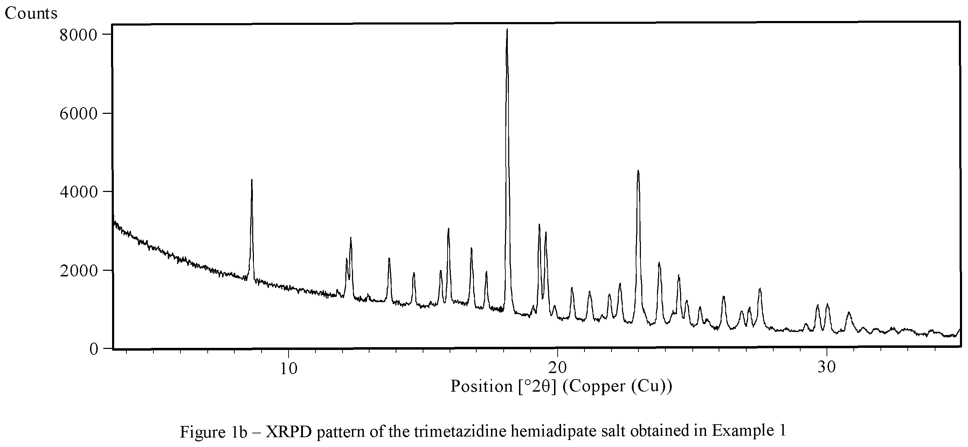

The corresponding XRPD pattern is in Figure 1b.

### EXAMPLE 2 : Trimetazidine hemisuccinate

### Process A.

5.0 g of trimetazidine and 1.11 g of succinic acid were placed in 75mL of ethanol. The mixture was placed under stirring at 70°C for 2 hours and then stirred for 60h at ambient temperature. The suspension was filtered, and the powder was dried at 40°C under vacuum overnight. The obtained powder corresponds to a crystalline powder.

¹H NMR: 6.96 ppm, 6.75 ppm, 3.77 ppm, 3.77 ppm, 3.73 ppm, 3.37 ppm, 2.80 ppm, 2.37 ppm, 2.26 ppm.

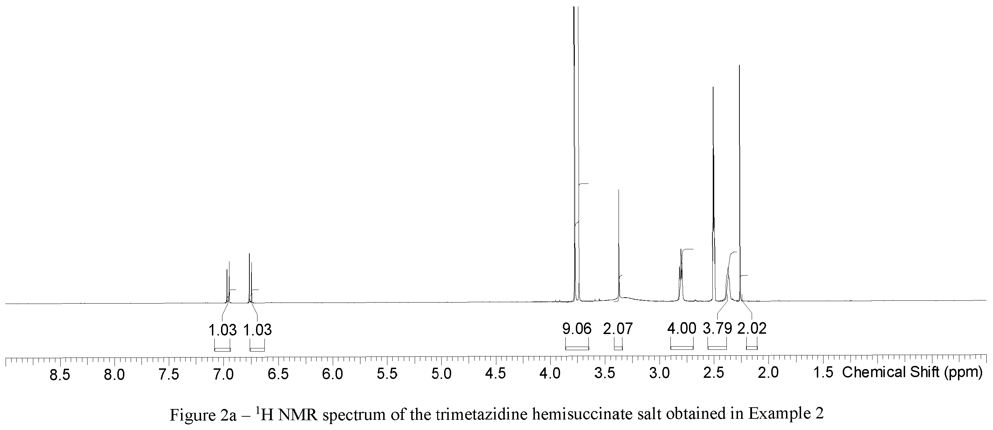

The corresponding ¹H NMR spectrum is in Figure 2a.

**XRPD table:**

| | Position [°2θ] | Relative Intensity [%] |
|---|---|---|
| 1 | 15.50 | 80 |
| 2 | 15.80 | 100 |
| 3 | 16.29 | 19 |
| 4 | 19.03 | 36 |
| 5 | 19.77 | 25 |
| 6 | 23.20 | 19 |
| 7 | 24.34 | 17 |
| 8 | 24.64 | 17 |
| 9 | 26.74 | 21 |

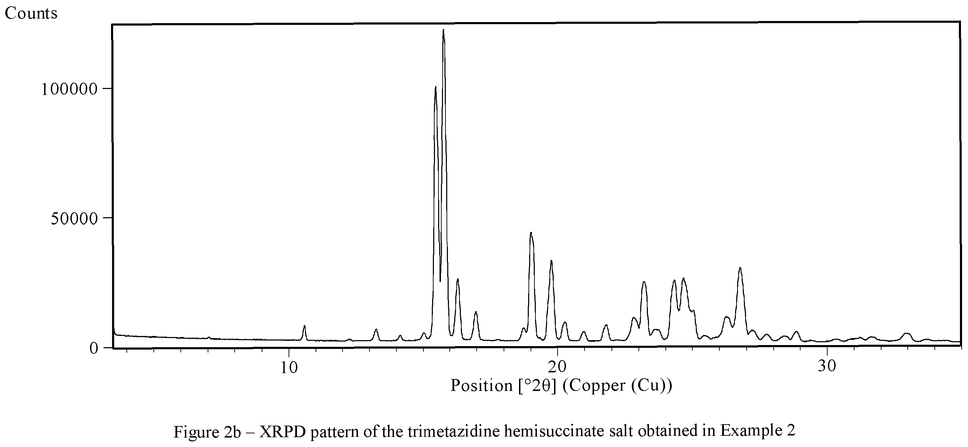

The corresponding XRPD pattern is in Figure 2b.

### Process B.

64kg of trimetazidine, 14kg of succinic acid , 4.9kg of water and 569kg of isopropyl alcohol were placed in a reactor.

The mixture was placed under mechanical stirring at 72°C for 30 minutes. This solution was cooled to 63°C with a rate of 0.3°C/min. After 60 minutes at 63°C and under stirring, the suspension was cooled to 5°C with a rate of 0.25°C/min. The suspension was filtered, and the powder was dried at 40°C overnight. The obtained powder corresponds to a crystalline powder. The yield is about 91%.

The XRPD pattern is the same as with Process A.

### EXAMPLE 3: Trimetazidine hemimalate

1.0 g of trimetazidine and 0.25 g of malic acid were placed in 4mL of isopropanol. The mixture was stirred for 22h at ambient temperature. The suspension was filtered, and the powder was dried at 40°C under vacuum overnight. The obtained powder corresponds to a crystalline powder.

¹H NMR: 6.96 ppm, 6.76 ppm, 3.77 ppm, 3.77 ppm, 3.73 ppm, 3.39 ppm, 2.85 ppm, 2.45 ppm, 2.40 ppm, 2.30 ppm.

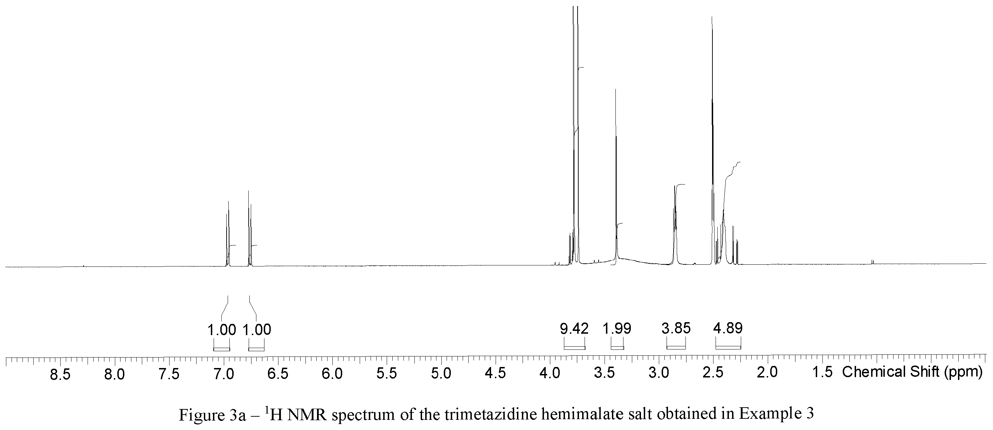

The corresponding ¹H NMR spectrum is in Figure 3a.

**XRPD table:**

| | Position [°2θ] | Relative Intensity [%] |
|---|---|---|
| 1 | 10.46 | 31 |
| 2 | 13.97 | 14 |
| 3 | 15.28 | 49 |
| 4 | 15.57 | 100 |
| 5 | 16.07 | 43 |
| 6 | 16.72 | 15 |
| 7 | 19.32 | 34 |
| 8 | 19.54 | 44 |
| 9 | 23.22 | 32 |
| 10 | 23.88 | 24 |
| 11 | 24.28 | 18 |
| 12 | 24.97 | 21 |
| 13 | 26.10 | 25 |
| 14 | 28.74 | 10 |

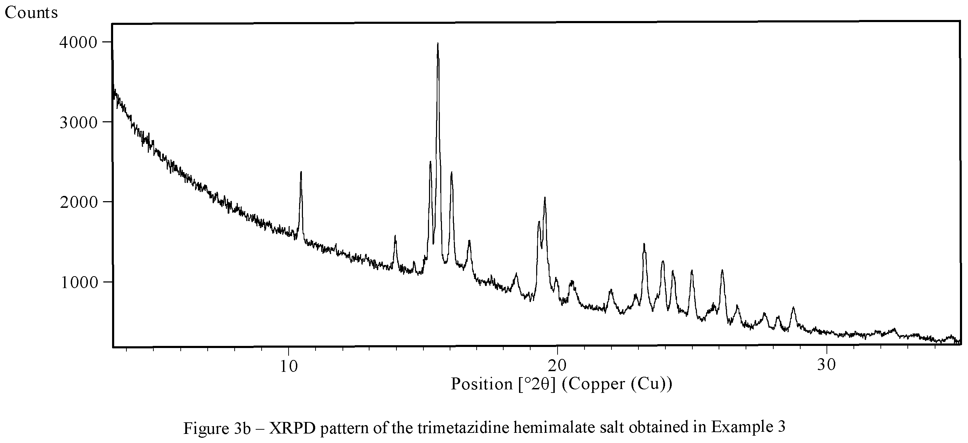

The corresponding XRPD pattern is in Figure 3b.

### EXAMPLE 4 : Trimetazidine hemisulfate

550.0 g of trimetazidine and 211 g of sulfuric acid solution at 96% were placed in 2.2 L of toluene. The mixture was stirred for 24h at 20°C. The suspension was filtered, and the powder was dried at 40°C under vacuum overnight. The obtained powder corresponds to a crystalline powder.

**XRPD table:**

| | Position [°2θ] | Relative Intensity [%] |
|---|---|---|
| 1 | 3.71 | 38 |
| 2 | 11.19 | 42 |
| 3 | 13.46 | 11 |
| 4 | 15.41 | 40 |
| 5 | 15.74 | 24 |
| 6 | 16.29 | 100 |
| 7 | 17.95 | 12 |
| 8 | 18.39 | 31 |
| 9 | 19.00 | 24 |
| 10 | 20.17 | 15 |
| 11 | 22.44 | 18 |
| 12 | 22.82 | 11 |
| 13 | 24.06 | 35 |
| 14 | 24.91 | 10 |
| 15 | 26.33 | 15 |
| 16 | 26.84 | 14 |

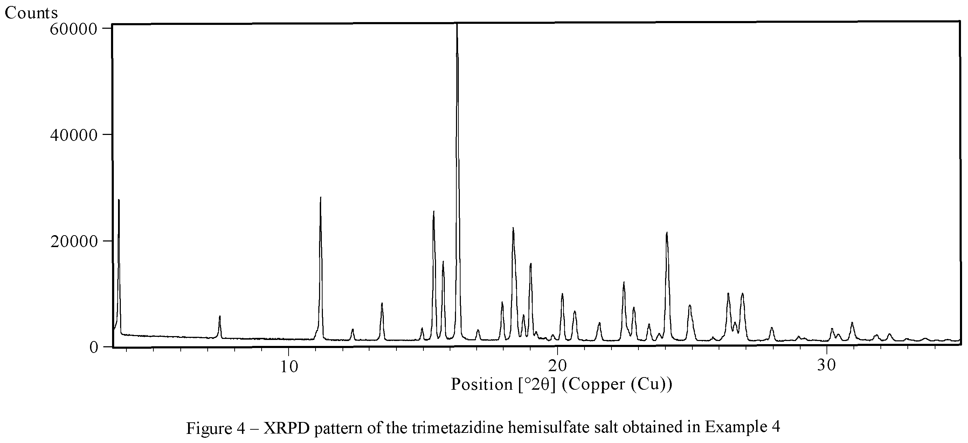

The corresponding X-Ray spectrum is in Figure 4.

### EXAMPLE 5 : Trimetazidine hemifumarate

1.0 g of trimetazidine and 0.23 g of fumaric acid were placed in 4mL of isopropanol. The mixture was stirred for 22h at ambient temperature. The suspension was filtered, and the powder was dried at 40°C under vacuum overnight. The obtained powder corresponds to a crystalline powder.

¹H NMR: 7.01 ppm, 6.76 ppm, 6.66 ppm, 3.87 ppm, 3.84 ppm, 3.82 ppm, 3.56 ppm, 3.12 ppm, 2.66 ppm.

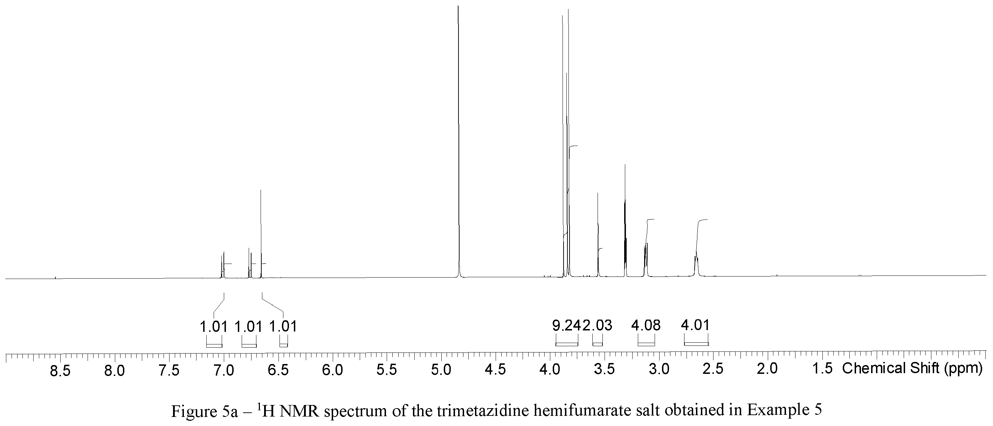

The corresponding ¹H NMR spectrum is in Figure 5a.

**XRPD table:**

| | Position [°2θ] | Relative Intensity [%] |
|---|---|---|
| 1 | 10.47 | 16 |
| 2 | 13.99 | 13 |
| 3 | 15.29 | 66 |
| 4 | 15.61 | 100 |
| 5 | 16.14 | 21 |
| 6 | 20.75 | 15 |
| 7 | 21.67 | 73 |
| 8 | 24.29 | 78 |

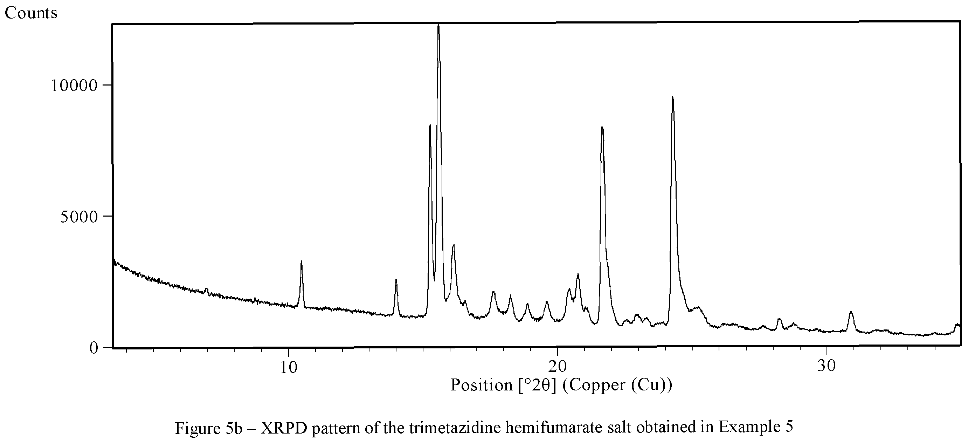

The corresponding XRPD pattern is in Figure 5b.

### EXAMPLE 6 : Trimetazidine hemitartrate

5.0 g of trimetazidine and 1.42 g of L-tartaric acid were placed in 75mL of ani sole. The mixture was placed under stirring at 70°C for 2 hours and then stirred for 60h at ambient temperature. The suspension was filtered, and the powder was dried at 40°C under vacuum overnight. The obtained powder corresponds to a crystalline powder.

¹H NMR: 6.96 ppm, 6.76 ppm, 3.77 ppm, 3.73 ppm, 3.39 ppm, 2.86 ppm, 2.41 ppm.

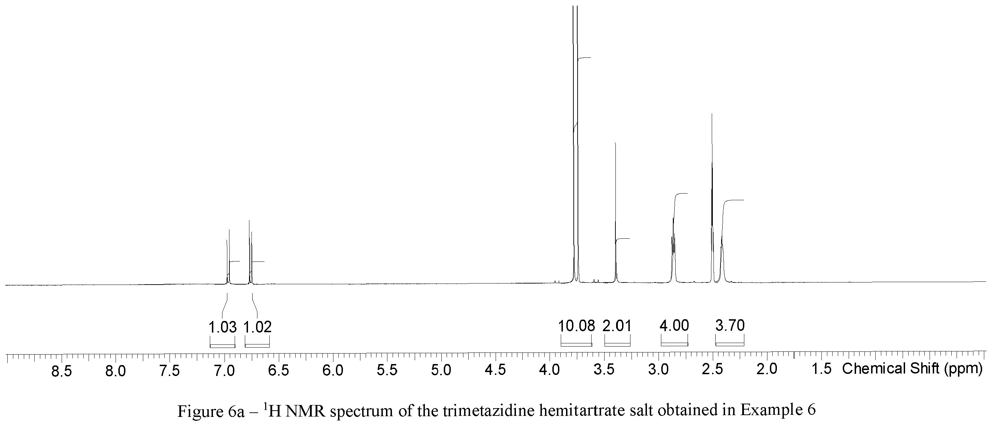

The corresponding ¹H NMR spectrum is in Figure 6a.

**XRPD table:**

| | Position [°2θ] | Relative Intensity [%] |
|---|---|---|
| 1 | 10.39 | 22 |
| 2 | 15.02 | 58 |
| 3 | 15.43 | 100 |
| 4 | 16.53 | 21 |
| 5 | 18.28 | 16 |
| 6 | 19.58 | 20 |
| 7 | 20.47 | 29 |
| 8 | 23.14 | 29 |
| 9 | 23.43 | 25 |
| 10 | 24.21 | 32 |
| 11 | 25.70 | 23 |

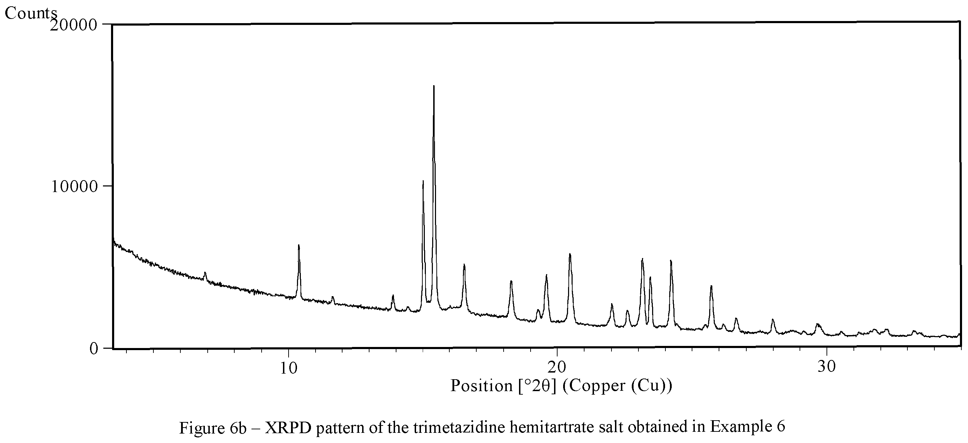

The corresponding XRPD pattern is in Figure 6b.

### EXAMPLE 7 : Trimetazidine hemiphosphate

1.0 g of trimetazidine and 0.18 g of phosphoric acid were placed in 4mL of isopropanol. The mixture was stirred for 22h at ambient temperature. The suspension was filtered, and the powder was dried at 40°C under vacuum overnight. The obtained powder corresponds to a crystalline powder.

**XRPD table:**

| | Position [°2θ] | Relative Intensity [%] |
|---|---|---|
| 1 | 3.58 | 23 |
| 2 | 10.78 | 29 |
| 3 | 13.65 | 11 |
| 4 | 14.56 | 27 |
| 5 | 15.21 | 18 |
| 6 | 15.87 | 35 |
| 7 | 16.01 | 91 |
| 8 | 16.23 | 49 |
| 9 | 16.37 | 31 |
| 10 | 16.90 | 100 |
| 11 | 17.08 | 22 |
| 12 | 17.54 | 15 |
| 13 | 23.67 | 12 |

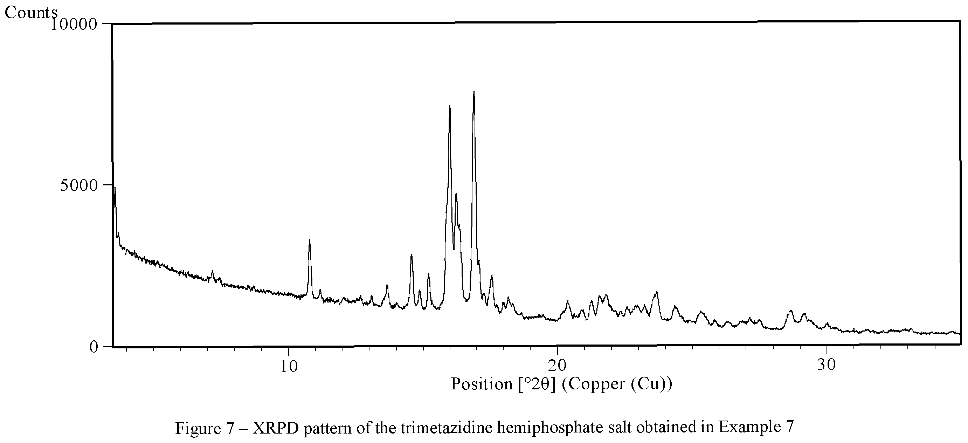

The corresponding XRPD pattern is in Figure 7.

### EXAMPLE 8: Comparative experiment: trimetazidine nitrosamine formation

In order to model the formation of trimetazidine nitrosamine during the formulation process, an aqueous solution of sodium nitrite was added to the trimetazidine salt. The sodium nitrite mimics the presence of nitrites in water and/or excipients during a drug product manufacturing process.

The trimetazidine salt, in an amount corresponding to 600 mg trimetazidine, was placed in 2 mL volumetric flask. 150 µL of sodium nitrite aqueous solution at 0.12mg/mL was placed in the 2mL volumetric flask and the volume was adjusted to 2mL with water to obtain a solution at 300 mg/mL in trimetazidine equivalent.

The solution was stirred at 40°C for 22 hours. The trimetazidine nitrosamine dosage was performed by LC/MS.

Due to the low solubilities of the dioxalate and oxalate trimetazidine salts, the experiments with these two salts were respectively performed at 6 mg/mL and 30 mg/mL instead of 300 mg/mL in trimetazidine equivalent.

### Results:

| Compound | Trimetazidine salt | Trimetazidine nitrosamine content (ppm) |
|---|---|---|
| Example 1 | Hemiadipate | 0.7 |
| Example 2 | Hemisuccinate | 0.5 |
| Example 3 | Hemimalate | 0.7 |
| Example 4 | Hemisulfate | 0.6 |
| Example 5 | Hemifumarate | 0.1 |
| Example 6 | Hemitartrate | 0.6 |
| Example 7 | Hemiphosphate | 0.6 |
| Comparative Example A | Dihydrochloride | 126.0 |
| Comparative Example B | Hemioxalate | 1.1 |
| Comparative Example C | Oxalate | 104.0 |
| Comparative Example D | Dioxalate | 83.1 |
| Comparative Example E | Hydrochloride | 22.0 |
| Comparative Example F | Maleate | 121.2 |
| Comparative Example G | Fumarate | 31.9 |

The above results show that the trimetazidine salts of the present invention perform better than the trimetazidine salts of the prior art.

### EXAMPLE 9 : Immediate release formulation - corresponding to a formulation containing 20 mg trimetazidine dihydrochloride

| | |
|---|---|
| Trimetazidine salt | 18.6 to 20.1 mg (see the table below) |
| Maize starch | 26 mg |
| Mannitol | 34 mg |
| Polyvidone | 4 mg |
| Magnesium stearate | 1 mg |
| Talc | 5 mg |

**Equivalence table**

| Compound | Trimetazidine salt | M (g/mol) | Amount of trimetazidine salt in the IR tablet formulation (mg) |
|---|---|---|---|
| Example 1 | Hemiadipate | 339.40 | 20.0 |
| Example 2 | Hemisuccinate | 325.38 | 19.2 |
| Example 3 | Hemimalate | 333.38 | 19.7 |
| Example 4 | Hemisulfate | 315.37 | 18.6 |
| Example 5 | Hemifumarate | 324.37 | 19.1 |
| Example 6 | Hemitartrate | 341.38 | 20.1 |
| Example 7 | Hemiphosphate | 315.33 | 18.6 |

### EXAMPLE 10 : MR matrix tablet - corresponding to the matrix MR tablet containing 35 mg trimetazidine dihydrochloride

| | |
|---|---|
| Trimetazidine salt | 32.5 to 35.2 mg (see the table below) |
| Hydroxypropyl | 74.0 mg |
| Povidone | 8.7 mg |
| Calcium hydrogen phosphate dihydrate | 80.9 mg |
| Magnesium stearate | 1.0 mg |
| Anhydrous colloidal | 0.4mg |

**Equivalence table**

| Compound | Trimetazidine salt | M (g/mol) | Amount of trimetazidine salt in the MR matrix tablet formulation (mg) |
|---|---|---|---|
| Example 1 | Hemiadipate | 339.40 | 35.0 |
| Example 2 | Hemisuccinate | 325.38 | 33.6 |
| Example 3 | Hemimalate | 333.38 | 34.4 |
| Example 4 | Hemisulfate | 315.37 | 32.5 |
| Example 5 | Hemifumarate | 324.37 | 33.5 |
| Example 6 | Hemitartrate | 341.38 | 35.2 |
| Example 7 | Hemiphosphate | 315.33 | 32.5 |

### EXAMPLE 11 : OD formulation - corresponding to the OD formulation containing 80 mg trimetazidine dihydrochloride

| | |
|---|---|
| Trimetazidine salt | 74.4 to 80.5 mg (see the table below) |
| Neutral sucrose/maize starch minigranules | 36.67 mg |
| Hydroxypropyl methylcellulose | 6.40 mg |
| Acetyl tributyl citrate | 1.20 mg |
| Ethylcellulose | 8.00 mg |
| Talc | 12.00 mg |
| Magnesium stearate | 0.43 mg |

**Equivalence table**

| Compound | Trimetazidine salt | M (g/mol) | Amount of trimetazidine salt in the OD formulation (mg) |
|---|---|---|---|
| Example 1 | Hemiadipate | 339.40 | 80.0 |
| Example 2 | Hemisuccinate | 325.38 | 76.7 |
| Example 3 | Hemimalate | 333.38 | 78.6 |
| Example 4 | Hemisulfate | 315.37 | 74.4 |
| Example 5 | Hemifumarate | 324.37 | 76.5 |
| Example 6 | Hemitartrate | 341.38 | 80.5 |
| Example 7 | Hemiphosphate | 315.33 | 74.4 |

### EXAMPLE 12: OD formulation- combination with bisoprolol hemifumarate

The trimetazidine salt minigranules obtained in Example 11 and bisoprolol hemifumarate pellets containing 5 mg bisoprolol hemifumarate are mixed together and encapsulated.

### EXAMPLE 13: OD formulation- combination with metoprolol succinate

The trimetazidine salt minigranules obtained in Example 11 and metoprolol succinate pellets containing either 47.5 mg, or 95 mg metoprolol succinate are mixed together and encapsulated.

## Claims

1. A trimetazidine salt which is selected from trimetazidine hemimalate, trimetazidine hemiadipate, trimetazidine hemitartrate, trimetazidine hemiphosphate, trimetazidine hemisulfate, trimetazidine hemisuccinate and trimetazidine hemifumarate, their hydrates, and crystalline forms thereof.

2. A process of synthesis of a trimetazidine salt according to Claim 1, wherein trimetazidine is reacted with an organic acid selected from malic acid, adipic acid, tartaric acid, phosphoric acid, sulfuric acid, succinic acid, and fumaric acid in a solvent, leading to the corresponding salt having a trimetazidine/organic acid ratio of 2/1.

3. A process according to Claim 2, wherein trimetazidine is reacted with 0.5 eq. malic acid, adipic acid, tartaric acid, phosphoric acid, sulfuric acid, succinic acid or fumaric acid, in a solvent, leading to the corresponding trimetazidine hemimalate, hemiadipate, hemitartrate, hemiphosphate, hemisulfate, hemisuccinate or hemifumarate having a trimetazidine/ malic acid, trimetazidine/ adipic acid, trimetazidine/ tartaric acid, trimetazidine/ phosphoric acid, trimetazidine/ sulfuric acid, trimetazidine/ succinic acid or trimetazidine/ fumaric acid molar ratio of 2/1.

4. A process according to anyone of Claims 2-3, wherein the solvent is selected from anisole, methyl isobutyl ketone, toluene, n-heptane, acetonitrile, methyl ethyl ketone, ethyl acetate, 1,3-dioxalane, tetrahydrofuran, acetone, methyl tert-butyl ether, water, methanol, ethanol, isopropanol, isobutanol, n-butanol, and mixtures thereof.

5. **A** pharmaceutical composition comprising a trimetazidine salt according to Claim 1, in combination with one or more inert, non-toxic, pharmaceutically acceptable excipients or carriers.

6. A pharmaceutical composition according to Claim 5, which is in the form of an immediate-release oral tablet, in the form of a prolonged release oral matric tablet or in the form of coated minigranules in capsules, for oral, once-a-day administration.

7. A pharmaceutical composition according to any one of Claims 5 or 6, further comprising a betablocker.

8. A pharmaceutical composition according to Claim 7, wherein the betablocker is metoprolol or bisoprolol.

9. A trimetazidine salt according to Claim 1 or a pharmaceutical composition according to any one of Claims 5 to 8, for use in the prophylactic treatment of angina pectoris, in the course of chorioretinal disorders or for the treatment of vertigo of vascular origin.

## Patentansprüche

1. Trimetazidinsalz, ausgewählt aus Trimetazidinhemimalat, Trimetazidinhemiadipat, Trimetazidinhemitartrat, Trimetazidinhemiphosphat, Trimetazidinhemisulfat, Trimetazidinhemisuccinat und Trimetazidinhemifumarat, deren Hydraten und kristallinen Formen davon.

2. Verfahren zur Synthese eines Trimetazidinsalzes nach Anspruch 1, wobei Trimetazidin mit einer organischen Säure, ausgewählt aus Äpfelsäure, Adipinsäure, Weinsäure, Phosphorsäure, Schwefelsäure, Bernsteinsäure und Fumarsäure, in einem Lösungsmittel umgesetzt wird, was zu dem entsprechenden Salz mit einem Verhältnis von Trimetazidin/organischer Säure von 2/1 führt.

3. Verfahren nach Anspruch 2, wobei Trimetazidin mit 0,5 Äq. Äpfelsäure, Adipinsäure, Weinsäure, Phosphorsäure, Schwefelsäure, Bernsteinsäure oder Fumarsäure in einem Lösungsmittel umgesetzt wird, was zu dem entsprechenden Trimetazidinhemimalat, -hemiadipat, -hemitartrat, -hemiphosphat, -hemisulfat, - hemisuccinat oder -hemifumarat mit einem Molverhältnis von Trimetazidin/Äpfelsäure, Trimetazidin/Adipinsäure, Trimetazidin/Weinsäure, Trimetazidin/Phosphorsäure, Trimetazidin/Schwefelsäure, Trimetazidin/Bernsteinsäure oder Trimetazidin/Fumarsäure von 2/1 führt.

4. Verfahren nach einem der Ansprüche 2-3, wobei das Lösungsmittel ausgewählt ist aus Anisol, Methylisobutylketon, Toluol, n-Heptan, Acetonitril, Methylethylketon, Ethylacetat, 1,3-Dioxalan, Tetrahydrofuran, Aceton, Methyl-tert-butylether, Wasser, Methanol, Ethanol, Isopropanol, Isobutanol, n-Butanol und Mischungen davon.

5. Pharmazeutische Zusammensetzung, umfassend ein Trimetazidinsalz nach Anspruch 1 in Kombination mit einem oder mehreren inerten, nicht-toxischen, pharmazeutisch verträglichen Hilfsstoffen oder Trägern.

6. Pharmazeutische Zusammensetzung nach Anspruch 5, die in Form einer oralen Tablette mit sofortiger Freisetzung, in Form einer Matrixtablette mit verlängerter Freisetzung oder in Form von beschichteten Minigranulaten in Kapseln zur oralen, einmal täglichen Verabreichung vorliegt.

7. Pharmazeutische Zusammensetzung nach einem der Ansprüche 5 oder 6, weiterhin umfassend einen Betablocker.

8. Pharmazeutische Zusammensetzung nach Anspruch 7, wobei der Betablocker Metoprolol oder Bisoprolol ist.

9. Trimetazidinsalz nach Anspruch 1 oder pharmazeutische Zusammensetzung nach einem der Ansprüche 5 bis 8 zur Verwendung bei der prophylaktischen Behandlung von Angina pectoris, im Verlauf von chorioretinalen Erkrankungen oder zur Behandlung von Schwindel vaskulären Ursprungs.

## Revendications

1. Sel de trimétazidine qui est choisi parmi l'hémimalate de trimétazidine, l'hémiadipate de trimétazidine, l'hémitartrate de trimétazidine, l'hémiphosphate de trimétazidine, l'hémisulfate de trimétazidine, l'hémisuccinate de trimétazidine et l'hémifumarate de trimétazidine, leurs hydrates et leurs formes cristallines.

2. Procédé de synthèse d'un sel de trimétazidine selon la revendication 1, dans lequel la trimétazidine est mise à réagir avec un acide organique choisi parmi l'acide malique, l'acide adipique, l'acide tartrique, l'acide phosphorique, l'acide sulfurique, l'acide succinique et l'acide fumarique dans un solvant, conduisant au sel correspondant ayant un rapport trimétazidine/acide organique de 2/1.

3. Procédé selon la revendication 2, dans lequel la trimétazidine est mise à réagir avec 0,5 éq. d'acide malique, d'acide adipique, d'acide tartrique, d'acide phosphorique, d'acide sulfurique, d'acide succinique ou d'acide fumarique, dans un solvant, conduisant à l'hémimalate, l'hémiadipate, l'hémitartrate, l'hémiphosphate, l'hémisulfate, l'hémisuccinate ou l'hémifumarate de trimétazidine correspondant ayant un rapport molaire trimétazidine/acide malique, trimétazidine/acide adipique, trimétazidine/acide tartrique, trimétazidine/acide phosphorique, trimétazidine/acide sulfurique, trimétazidine/acide succinique ou trimétazidine/acide fumarique de 2/1.

4. Procédé selon l'une quelconque des revendications 2 à 3, dans lequel le solvant est choisi parmi l'anisole, la méthylisobutylcétone, le toluène, le n-heptane, l'acétonitrile, la méthyléthylcétone, l'acétate d'éthyle, le 1,3-dioxalane, le tétrahydrofurane, l'acétone, l'éther méthylique ter-butylique, l'eau, le méthanol, l'éthanol, l'isopropanol, l'isobutanol, le n-butanol et des mélanges de ceux-ci.

5. Composition pharmaceutique comprenant un sel de trimétazidine selon la revendication 1, en association avec un ou plusieurs excipients ou vecteurs pharmaceutiquement acceptables, non toxiques, inertes.

6. Composition pharmaceutique selon la revendication 5, se présentant sous forme de comprimé oral à libération immédiate, sous forme de comprimé matriciel oral à libération prolongée ou sous forme de minigranulés enrobés dans des capsules, pour une administration orale une fois par jour.

7. Composition pharmaceutique selon l'une quelconque des revendications 5 ou 6, comprenant également un bêtabloquant.

8. Composition pharmaceutique selon la revendication 7, dans laquelle le bêtabloquant est le métoprolol ou le bisoprolol.

9. Sel de trimétazidine selon la revendication 1 ou composition pharmaceutique selon l'une quelconque des revendications 5 à 8, pour une utilisation dans le traitement prophylactique de l'angine de poitrine, durant des troubles choriorétiniens ou pour le traitement des vertiges d'origine vasculaire.
